(19) Europäisches Patentamt
European Patent Office
Office européen des brevets

(11) Publication number: **0 034 126**
**B1**

(12) **EUROPEAN PATENT SPECIFICATION**

(45) Date of publication of patent specification: **04.07.84**

(51) Int. Cl.³: **A 61 K 7/15**

(21) Application number: **81830012.1**

(22) Date of filing: **23.01.81**

(54) **Cosmetic and dermatological compositions for shaving the skin.**

(30) Priority: **25.01.80 IT 1947580**

(43) Date of publication of application:
**19.08.81 Bulletin 81/33**

(45) Publication of the grant of the patent:
**04.07.84 Bulletin 84/27**

(84) Designated Contracting States:
**AT BE CH DE FR GB LI LU NL SE**

(56) References cited:
**FR - A - 2 080 759**
**FR - A - 2 383 660**
**GB - A - 1 479 707**
**GB - A - 1 479 708**
**US - A - 3 734 858**
**US - A - 3 855 290**

**SEIFEN-ÖLE-FETTE-WACHSE, vol. 99, no. 12, 01.06.1973, AUGSBURG (DE), G.P. LAUHUS: "Gafquat-Copolymere, eine neue Generation kationaktiver Harze für die Haarkosmetik", pages 333-337**

(73) Proprietor: **Maggesi, Luigi**
**33, Via Teodosio**
**I-20131 Milano (IT)**

(72) Inventor: **Maggesi, Luigi**
**33, Via Teodosio**
**I-20131 Milano (IT)**

(74) Representative: **Gervasi, Gemma et al,**
**Studio Brevetti e Marchi NOTARBARTOLO & GERVASI 33, Viale Bianca Maria**
**I-20122 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 034 126 B1**

Courier Press, Leamington Spa, England.

**Description**

This invention relates to compositions of cosmetic and dermatological action for shaving the skin.

The term "shaving the skin" is intended to indicate all those operations normally carried out on the human person in shaving the beard, moustache, and head and body hair, for aesthetic purposes, or for therapeutic purposes where it is necessary to completely expose skin zones which are wounded or on which surgical operations have to be carried out.

Shaving can be carried out by means of manual razors or electric shavers, with the aid of numerous cosmetic products at present available, and which can be grouped as follows:

1) shaving soaps in various forms (creams, liquids, sticks, lotions, foams etc.) constituted essentially by mixtures of $C_{12}$—$C_{18}$ fatty acid salts; these are generally alkaline salts and/or simple or ethoxylated aliphatic amines. These soaps are always more or less alkaline, and produce an abundant foam which wets and softens the hair, so facilitating its shaving by manual razors;

2) modified vanishing creams constituted essentially by emulsifying agents, vegegable oils, mineral oils, waxes and the like. These creams are rubbed on to the skin before manual shaving, and act as softeners of the keratins. However, they have a wetting power which is less than that of soaps;

3) lotions, powders and creams to be applied to the face before electric shaving, in that they favour the sliding of the shaving heads over the skin and thus facilitate shaving. These products generally contain vegetable oils, or their chemically reconstituted esters such as isopropylmyristate, isopropyldodecanol and the like in a hydroalcoholic carrier (lotions), or in an emulsified carrier (cream and milks). The powders are mainly talc-based.

Although all the products indicated under points 1, 2, 3 are certainly useful in facilitating shaving, in no case do they prevent skin irritation with its consequent reddening and crumbling. They also often form abrasions and small wounds, which are very annoying and can also be dangerous from the point of view of infection, especially during the shaving of acne or hyperseborrhoeic skins, or in the case of shaving parts already comprising wounds. The danger of infection is especially real where soaps of point (1) are used, in that their alkalinity (pH 8.5—9.5) alters the acid pH of the skin covering and thus eliminates or strongly reduces the greatest natural epidermis defence factor against external agents, and in particular against micro-organisms. In addition, shaving soaps and foams remove the cutaneous hydrolipid film which, as known, constitutes one of the most valid natural defences of the skin. Also products containing surfactants different from soaps are often alkaline in fact (pH about 8) as those described in FR—A—2 080 759.

In seeking to lessen the drawbacks due to shaving with the "shaving soaps" described under the preceding points, many "pre-shave" and "after-shave" products have been marketed. "Pre-shave" products generally contain hair softening substances and substances which facilitate the sliding of the razor, such as vaseline oil, vegetable oils etc. Products of this type are described for instance in FR—A—2 383 660, which products are based on a specific combination of anionic and cationic surfactants.

"After-shave" products are slightly antiseptic hydroalcoholic lotions which confer softness and perfume to the skin and essentially comprise water-soluble fatty esters, glycerine, glycols etc.

However, even the use of "pre-shave" or "after-shave" products does not succeed in eliminating the main drawbacks of shaving, i.e. abrasions and small surface wounds, skin irritation and the danger of infection. In addition, pre-shave and after-shave products are generally costly.

It has now been found, and forms the subject matter of the present invention, that it is possible to prepare completely new shaving aid compositions which combine all the cosmetic and dermatological functions which up to now have been performed by soaps, and pre-shave and after-shave products, and solving all problems which so far have remained unsolved by these products but without presenting any of the drawbacks thereof. The new shaving aid products are based on completely new concepts. In this respect, a class of compounds has been unexpectedly identified which when applied to the skin form thereon a thin protective lipophilic film on which the razor operates during shaving instead of directly on the skin. This film can adhere to a greater or lesser extent according to the nature and molecular complexity of the compounds used, but in all cases it prevents direct contact between the blade and skin, and thus prevents the damage which this contact normally causes.

The new components which, when used in critical quantities, characterise the new compositions of the present invention, all pertain to the class consisting of cationic organic compounds containing at least one quaternary ammonium nitrogen.

These compounds, which are able to perform said function which makes them unique shaving aids at pH 2—7, are preferably used in compositions comprising various components in various proportions, according to the type of product prepared.

The new shaving products comprise generally the following main components:

1) Cationic organic compounds containing at least one quaternary ammonium nitrogen
2) Animal and/or vegetable proteins and/or derivatives of proteins selected from the group of: soluble native collagen from animal tissues, desamido collagen, collagen complexed with cetyl-trimethyl ammonium-chloride, hydrolized animal proteins, protein hydrating products, hydrolyzed soya and milk proteins, protein derivatives from connective tissues of bovine source

3) Non-ionic and ampholitic surface active agents
4) Wetting, hydrating and supergreasing agents
5) Protective and gelling colloids
6) Preservatives and anti-mildew agents
7) Anti-foaming agents
8) Lubricating and anti-delipidising agents.

of which the components 1 to 4 are essential to obtain highly useful products, while components 5 through 8 are only optional additives for the preparation of compositions in specific form and with specific utility.

Where necessary, the products are suitably diluted, supported, coloured and perfumed by the normal substances for this purpose.

The new compositions are in any case free of soaps, of anionic polymers and anionic surfactants which, beside to chemically interfer with the quaternary ammonium compounds, alter the naturally acid pH of the skin covering, and remove the cutaneous hydrolipid film.

The content of said classes of products either essential or optional, for the present invention will now be considered in detail.

1. *Cationic organic compounds containing at least one quaternary ammonium nitrogen.*

These compounds constitute a well identified homogeneous class, and can be grouped from the chemical aspect into certain groups of compounds of closely analogous structure:

a) nitrogenised cationic organic compounds containing at least one quaternary ammonium group of formula:

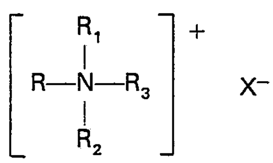

in which $R_1$ and $R_2$, which can be equal or different, are linear or branched alkyls of 1—20 C atoms, hydroxyalkyl of 1—20 C atoms, polyoxyalkylated chains of 2—40 C atoms, or hydroxypolyoxyalkylene chains of 2—40 C atoms;

R and $R_3$, which may be equal or different, are linear or branched alkyls of 1—20 C atoms, alkylaryls of 6—20 C atoms, polyoxyalkylene chains of 4—40 C atoms, hydroxypolyoxyalkylene chains of 4—40 C atoms; alkylamido groups with the terminal amido group of 2—20 C atoms, or amido-polyhydroxyalkylene chains of 6—20 C atoms;

X is an anion chosen from the group consisting of $OH^-$, halogen, sulphate, ethoxysulphate, nitrate, sulphite, acid carbonate, citrate, lactate, phosphate, tartrate, maleate, adipate or glutamate ions.

b) nitrogenised heterocyclic compounds, possibly containing other heteroatoms, and with one or two nitrogen substituents, contained within the general formula

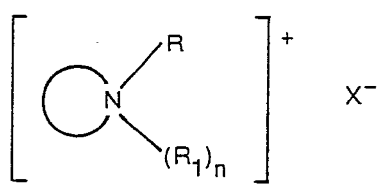

in which R is a linear or branched alkyl radical of 1—20 C atoms or a hydroxyalkyl radical of 1—20 C atoms; $R_1$ is an alkyl radical of 1—20 C atoms, a hydroxy alkyl radical of 1—20 C atoms, or aliphatic chains containing amido and/or ester groups of 4—40 C atoms;

n is 0 or 1

X is an anion as defined under point (a)

c) cationic organic polymers containing quaternary nitrogen atoms.

Polymers comprising units of the type

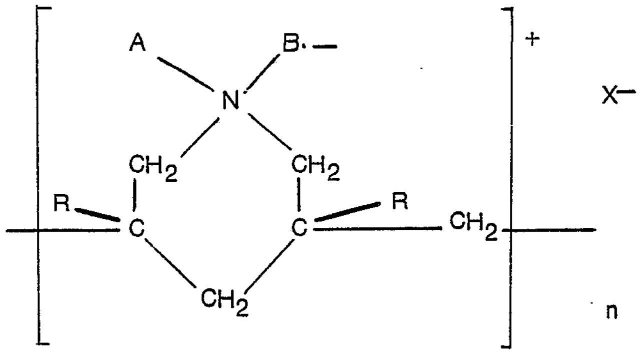

in which A and B, which can be equal or different, are hydrogen provided they are not both hydrogen, alkyl of 1—20 C atoms, hydroxyalkyl of 1—5 C atoms, alkylamido chains of 1—5 C atoms, or A and B together with the nitrogen to which they are bonded form a piperidine or morpholine ring;

R is hydrogen, an alkyl radical of 1—20 C atoms, a hydroxyalkyl radical of 1—5 C atoms, or an alkylamido radical of 1—5 C atoms;

$X^-$ is an anion as defined under point a).

The polymers containing the said units can be homopolymers or copolymers, preferably with acrylamide and diacetoneacrylamide, of molecular weight between 20,000 and 3,000,000.

The preparation of these polymers is described in USA patents 2,926,161, 3,288,770, 3,412,019.

d) cationic organic polymers of the type

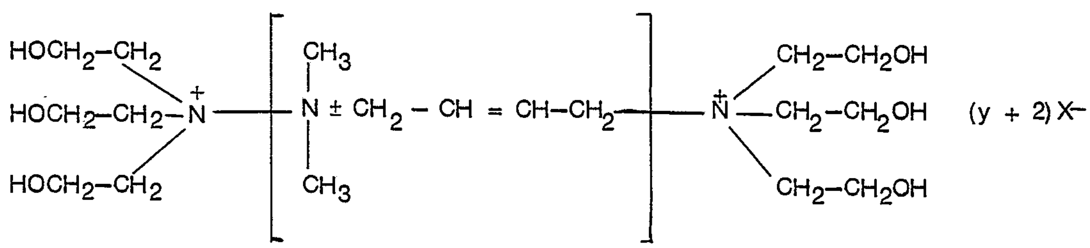

where $X^-$ is an anion as defined under point a);

y is 10—12.

The preparation of these polymers is described in USA patent 4,270,000. A product of this type is Onamer M (in which X is chloride) of Onyx-Millmaster U.S.A.

e) quaternised celluloses

i.e. celluloses of the type:

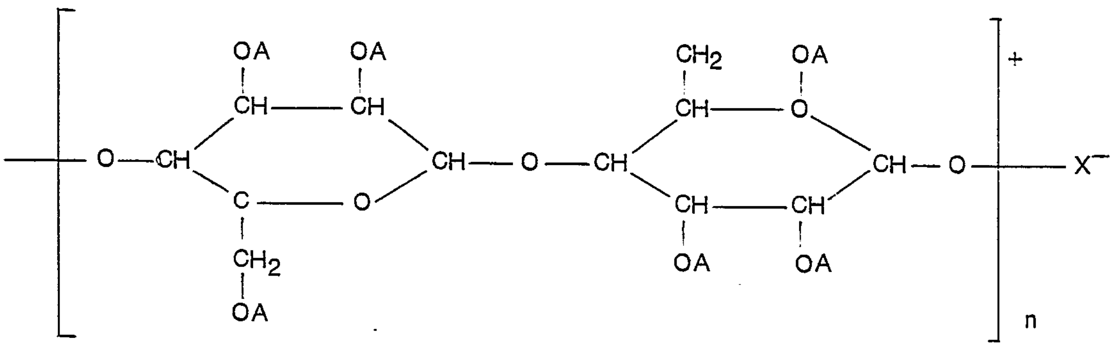

in which A is H or aliphatic radicals comprising a terminal quaternary ammonium group;

$X^-$ is as defined under point a).

Quaternised celluloses are prepared for example in accordance with USA patents 3,876,760 and 3,472,840 ("Polymer" series of Union Carbide USA)

f) quaternised copolymers of adipic acid such as copolymers of formula:

f1) 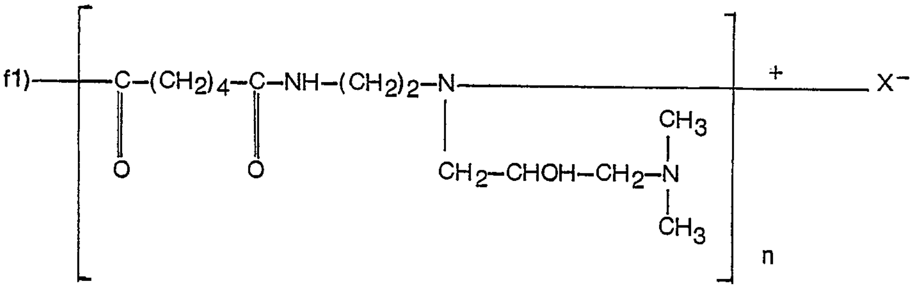

where n is 10—12 and X is as defined under point a).

A product of this type is marketed by Sandoz (USA) under the trademark Cartaretin F—4 and F—6.

f2) 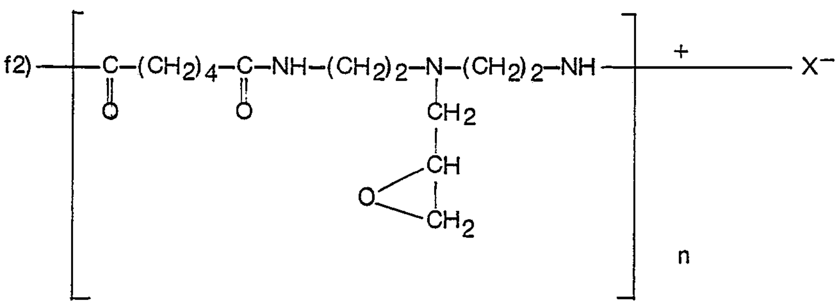

where n is 10—12 and X is an anion defined under point a).

A product of this type is marketed by Hercules (USA) under the trademark Delsette 101.

g) copolymers of acrylamide and $\beta$-methacrylyl-oxyethyltrimethyl ammonium chloride defined as Quaternium 39 by the C.T.F.A. Cosmetic Ingredients Dictionary published by Cosmetic Toiletry, Fragrance Association, 1133 Fifteenth Street, N.W., Washington (U.S.A.).

Products of this type are marketed by Hercules under the trademarks Reten 205, 210, 220;

h) guar-hydroxypropyl-trimethylammonium chloride marketed by General Mills (U.S.A.) as Cosmedia-Guar 21. "Guar" is exactly defined by de Navarre (see hereinafter) as among the hydrocolloids and natural gum cited hereinafter.

i) products of reaction between clays and quaternary ammonium salts of formula:

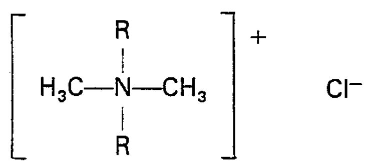

in which R represents fatty acid radicals of 12—24 C atoms.

Products of this type are marketed by NL Industries (U.S.A.) under the trademarks Bentone 27, 34 and 38, designated and defined by the C.T.F.A. Cosmetic Ingredients Dictionary as Stearalkonium-Hectorite, Quaternium 18-Bentonite and Quaternium 18-Hectorite.

Quaternary ammonium compounds preferred executing the present invention are the following:

Alkyl-($C_{10}$—$C_{20}$)-dimethyl-benzylammonium saccharinate (or chloride or bromide); Alkyl-($C_{12}$—$C_{14}$)-oxyethylene-(10)-ammonium phosphate; Behenyl-trimethyl-ammonium chloride; Cartaretin F4; Cetyl-dimethyl-hydroxyethyl-ammonium chloride; Cetyl-trimethyl-ammonium chloride; Cocoyl-amido-propyl-dimethyl-acetamido-ammonium chloride; Acrylamide/poly-(dimethyl-diallyl)-ammonium chloride copolymer; Crotein Q (quaternised protein); Delsette 101; Didecyl-dimethyl ammonium chloride; Diisobutyl-cresoxyethoxy-ethyl-dimethyl-benzyl ammonium chloride; Diisobutylphenoxy-ethoxy-ethyl-dimethyl-benzyl ammonium chloride; Dilauryl-dimethyl ammonium chloride; Dioctyl-dimethyl chloride; Distearyl-dimethyl ammonium chloride; Dodecyl-benzyl-triethanolammonium chloride; Dodecyl-dimethyl-ethylbenzyl ammonium chloride; $\delta$-gluconamido-propyl-dimethyl-2-hydroxyethyl ammonium chloride; quaternised Guar; quaternised hydroxyethyl-cellulose; Lauryl-dimethyl-benzyl ammonium chloride; N-(lauryl-cholamino-formyl-methyl)pyridine chloride; N-lauryl-N-ethyl-morpholine-ethoxysulphate; Lauryl-isoquinoline bromide; Lauryl-isoquinoline chloride; Lauryl-pyridine chloride; Lauryl-trimethyl ammonium chloride; Myristyl-amidopropyl-diethyl-2-hydroxyethyl ammonium chloride; Myristyl-pyridine chloride; Myristyl-dimethyl-ethylbenzyl-ammonium-cyclohexyl-sulphamate; Octyl-decyl-dimethyl ammonium chloride; Octyl-decyl-dimethyl ammonium chloride; Poly-(dimethyl-butenyl-ammonium chloride)-$\alpha$-$\omega$-bis-(triethanolammonium) chloride (Onamer M); Poly-(dimethyldiallyl)-

ammonium chloride; Polyoxypropylene (9) methyl-diethyl ammonium chloride; quaternised polyvinylpyrrolidone; Quaternium 18-Bentonite; Quaternium 18-Hectorite; Reten 205; Reten 210; Reten 220; N-(stearyl-choloamino-formyl-methyl)-pyridine chloride; Stearyl-dimethyl-benzylammonium chloride; Stearyl-dimethyl-hydroxyethylammonium chloride; Stearyl-imidazoline-dioxyethyl-ammonium chloride; Stearyl-pentaoxyethyl-ammonium chloride; Stearyl-triethanolammonium chloride; Trimethyl-dodecylbenzyl ammonium chloride; Catrex; Quaternum 27; Quaternum 29; Quaternum 54.

2. *Animal and/or vegetable proteins and/or protein hydrolysis, condensation or chemical transformation derivatives*

Among the numerous derivatives of natural proteins for cosmetic use at present available, the following types were selected and found useful for the present invention:

a) soluble native collagen extracted from various animal tissues.

Products of this type are marketed under the name Collapur of Freudeberg, Germany, Collagen of CLR-Richter, Germany, Pancogene S of Gattefosse, France, Embrio Collagen of Esperis, Italy and Collagenon of Vevy, Italy;

b) hydrolysed animal proteins (collagen, elastin, keratins, etc.).

Products of this type are marketed under the trademarks Hydrolised Cheratin M and Elastin of Freudeberg, Germany, Crotein A, ASC and Q of Croda, Great Britain; Lexein X250 and X1000 of Inolex, USA; Polypeptide LSN of Stepan, USA;

c) protein hydrating products.

Products of this type are marketed under the name Hydroplex HHR of CLR-Richter, Germany, Idrooutina of Croda Italiana, Lactil of Goldschmidt, Germany, Hydroviton of Dragoce, Germany;

d) hydrolised soya and milk proteins.

Products of this type are sold for example by Inolex (USA) under the trademarks Lexein and Lacto-Pro.

e) protein derivatives from connective tissue of bovine source;

f) collagen complexed with cetyl-trimethyl ammonium-chloride;

g) desamido-collagen

3. *Non-ionic and ampholitic surface active agents* —

Classes of surface active agents particularly suitable for the purposes of the present invention are:

— polyoxyethylene ethers of formula:

$$R—(OCH_2CH_2)_n—OR'$$

in which R is an alkyl or acyl of 4—32 C atoms, preferably 10—18 atoms, benzyl, naphthyl or $R'—C_6H_4$;

R' is H, an alkyl of 1—30 C atoms, a sulphuric or sulphonic group which can be salified with alkaline metals or simple or ethoxylated amines;

n is 1—40.

— polyoxyethylene-oxypropylene ethers of formula:

$$HO—(CH_2—CH_2—O)_x—(\underset{\displaystyle CH_3}{\underset{|}{C}H}—CH_2—O)_y—(CH_2—O)_z \quad H$$

in which x+y+z is 20—400.

— polyoxyethylenated sorbates such as polyoxyethylene (20) sorbitanmonolaurate, polyoxyethylene (20) sorbitanmonooleate, or polyoxyethylene (20) sorbitantrioleate;

— glyceric esters of fatty acids $C_8—C_{30}$

— polyoxyethylenated aliphatic amides of general formula

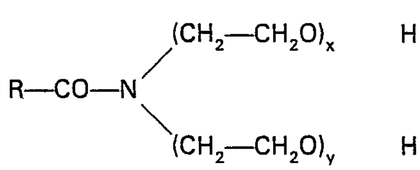

in which R is a saturated or unsaturated aliphatic radical of 8—40 C atoms, x+y is 1—20;

— polyoxyethylenated amines of general formula

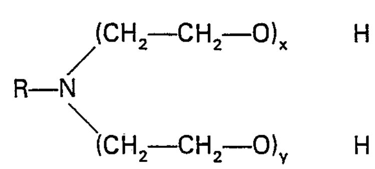

in which R is a saturated or unsaturated aliphatic or amino-aliphatic radical of 8—40 C atoms, and x+y is 2—30.

— polyoxyethylenated polyamines of general formula

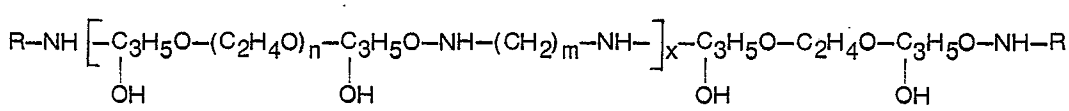

in which R is an alkyl radical of 12—24 C atoms, n is 10—20, m is 2—6, x is 2—4.

— amino oxides of formula

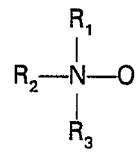

in which $R_1$ is a $C_6$—$C_{30}$ alkyl, preferably $C_{12}$—$C_{16}$, or

$R_1CO$—NM—$(CH_2)_a$— where a is 2—8

$R_2$, $R_3$ are $C_1$—$C_6$ alkyl or hydroxyalkyl radicals, or together with the nitrogen atom to which they are bonded can form a heterocyclic six element ring, possibly containing other heteroatoms.

Typical examples of these compounds are myristyl-dimethylamine oxide, bis-hydroxyethyl-laurylamine oxide, palmityl-dimethylamine oxide, myristyl-methyl-butylamine oxide, and amido-($C_{12}$—$C_{16}$)-amine oxide;

— polyoxyethylene glycol esters-(2—100) of fatty acids $C_8$—$C_{30}$

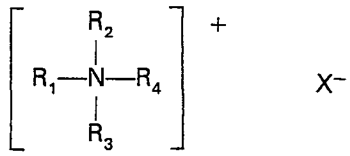

in which

X is $SO_3^{-2}$, $COO^-$

$R_1$ is a $C_8$—$C_{18}$ alkyl or R'—CO—NH—$(CH_2)_3$— in which R' is a $C_{10}$—$C_{20}$ alkyl radical

$R_2$ and $R_3$, which can be equal or different, are $C_1$—$C_4$ alkyls

$R_4$ is an alkylene or oxyalkylene radical of 1—4 C atoms

— imidazoline derivatives of formula

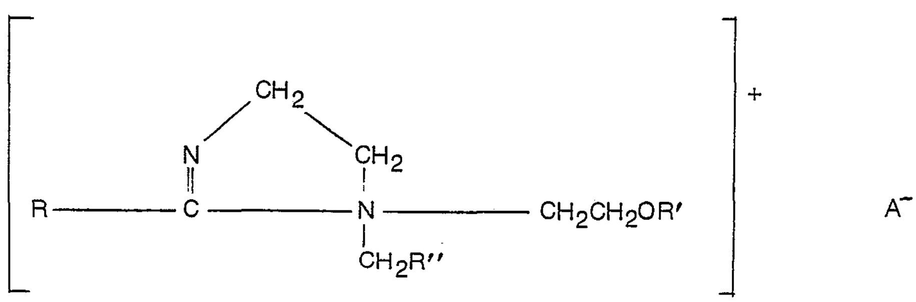

in which

R is a $C_6$—$C_{20}$ organic fatty radical

R' is H or a —$CH_2$—COOM group

R'' is —COOM, —$CH_2$COOM, —CHOH—$CH_2SO_3$M, —$CH_2$—O—$CH_2$—COON or R-substituted oxyethylimidazoline

A is hydroxyl, chloride, —$SO_4$M or —$OSO_3$M

M is sodium, H or a nitrogenised organic radical

4. *Wetting, hydrating and supergreasing agents*

These components of the new compositions are chosen from the following classes of compounds:

— polyethers of formula

$$H—(O—CH_2—CH_2)_n—OH$$

in which n is a whole member from 1 to 150

— polyglycols of formula

$$R—(CH—OH)_n—CH_2OH$$

in which R is a hydrogen or an alkyl of 1—6 C atoms, and n is a whole number from 2 to 100.
— 2-pyrrolidon carboxylic acid and derivatives thereof
— lanolin and its derivatives such as lanolin oils, lanolin alcohols, acetylated lanolin alcohols, polyoxyethylenated lanolin alcohols, lanolin waxes and their derivatives, mixtures of lanolin with vaseline oil and the like, lanolin esters and hydrogenated lanolin esters, lanolin polyoxyethylene ethers, quaternised lanolin.

5. *Protecting and gelling colloids*
These components of the new compositions according to the present invention are chosen from the following groups of compounds: polyvinylpyrrolidone and its derivatives, celluloses and their derivatives. Moreover, gums (hydrocolloids) and similar materials (with the exclusion of any material which may be anionic type) can advantageously be used as described and defined by Maison G. de Navarre in "The Chemistry and Manufacture of Cosmetics" vol. II, Cosmetic Materials, page 109 to 154, published by Van Nostrand U.S.A., 1962.
6. *Preservatives and anti-mildew agents* particularly suitable for the purposes of the present invention are p. oxybenzoic ester, sorbic ester, dehydroacetic ester, imidazoline, ureas and derivatives, E.D.T.A. and its acids salts of sodium, potassium and ammonium etc.
7. *Preferred anti-foaming agents* are those based on silicons.
8. *Lubricants and anti-delipidising agents* advantageously used in the present invention are:
— oxyethylenated derivatives of propylene oxide of formula:

$$R—(O—CH_2—\underset{\displaystyle CH_3}{\underset{|}{CH}})_x(O—CH_2—CH_2)_y—OH$$

in which
R is H or a $C_1$—$C_4$ alkyl, x is a whole number between 2 and 33, y is 0—45.
— saturated mineral oils of specific gravity between 0.831 and 0.875; vegetable oils, ethoxylated, propoxylated and polyoxyethylated vegetable oils;
—esters of simple or hydrogenated $C_6$—$C_{30}$ fatty acids and/or fatty oxyacids with $C_6$—$C_{18}$ alcohols and their water-soluble derivatives.

Of these, the most significant and useful for the present invention have proved to be: isopropylmyristate, isopropylstearate, diisopropyladipate, hexyllaurate, polyethyleneglyceryl-monolaurate, decyloleate, octadecanol, tetradecyl-octadodecanol, polyoxyethyleneglycol (200) monooctanoate, stearyl-heptanoate, cetylstearyl-2-ethylhexanoate, capryl-capric glycerides of polyoxyethylene (6), polyoxpropylene-glycol-(15)-stearylether, glyceryl stearate, hexyl laurate, polyethylenglycol glyceryl monococoate.
9. *The solvents and/or dispersing and/or solubilising agents* must generally be present to give greater uniformity of the finished product. They have been chosen from the following compounds: water, ethanol, isopropanol, benzyl alcohol, glycols and polyglycols, glycerines and polyvalent alcohols, hexanol, hexane, cyclohexane, furfurol, glycolethers (such as diethyleneglycol-monoethylether, diethyleneglycol-monomethylether, diethyleneglycol-monobutylether, (1,2)-propanediol-monobutylether, polyoxypropylene-glycol-(2,100)-methylether (and/or ethylether), ethyleneglycol-monomethylether (and/or monoethyl), ethyleneglycol-monobutylether, ethyleneglycol-monoethylether acetilate), butanols (and in particular ter-butanol), (1,5)-pentanediol, 2-ethoxyethanol, 2-phenoxyethanol, 1-butoxy-ethoxy-2-propanol, polypropyleneglycol-2-methylether and the like. All these compounds are defined in CTFA Cosmetic Dictionary.

The minimum quantity of such substances necessary to ensure homogeneousness varies within very wide limits depending on the type and quantity of the other ingredients present.

The inert, diluent or filler materials are substances added as complementary agents for forming a suitable base for correctly carrying the necessary active principles.

All the components indicated briefly heretofore are present in the new compositions in precise and critical proportions as specified hereinafter:

| | |
|---|---|
| 1 — Cationic organic compounds | 0.5—25%<br>preferably 2—5% |
| 2 — Proteins or protein derivatives | 1—20%<br>preferably 1—2% |
| 3 — Surface active agents | 0.01—15%<br>preferably 1—3% |

| | |
|---|---|
| 4 — Wetting and hydrating agents | 1—50% preferably 5—20% |
| 5 — Protecting and gelling colloids | 0.5—10% preferably 0.5—1.5% |
| 6 — Preservatives, anti-mildew agents | 0.05—3% preferably 0.1—1% |
| 7 — Anti-foaming agents | 0.05—3% preferably 0.1—1% |
| 8 — Lubricants and Antidelipidising agents | 0.5—20% preferably 5—20% |
| 9 — Solvents and/or dispersing agents and/or solubilising agents and/or inert; diluent, filler, perfume and colouring materials | quantity necessary to make up to 100 |

If required, other specific components can be added to these for forming shaving products to which special properties are to be given, such as bacteriostatic activity, fungicide activity, decongestioning activity, anti-acne action, wound healing action etc.

For example, small percentages of anaesthetics, sulphamides, antibiotics, vitamines, keratolythic, keratoplastic and other substances can be added. The pH of the product according to the present invention must be between 2 and 7, and must be preferably adjusted to between 4 and 6 by organic and inorganic acids, the anion of which is the $X^-$ as described in a) for the quaternary ammonium salts, or with alkalis of the sodium hydroxide type, triethanolamine and the like.

As initially stated, the new shaving aids according to the present invention are based on completely new concepts. In particular, they are based on the fact that the cationic organic compounds (I) at pH 2—7 form a thin protective lipophilic film on the skin on which the razor operates, instead of directly on the skin during shaving.

The formation of the protective layer on the skin is aided by the proteins (2) which become weakly fixed to the cationic layer by their chemical bonds, and the protective colloids (5). In addition, both the cationic compounds (1) and the protective colloids (5) have demonstrated a high capacity for bonding water molecules to the keratinic structure, in that they probably cause opening of the alpha-helixes which comprise them, and thus make them softer and easier to cut. This function is improved by the wetting and hydrating products (4), which also adjust the hydration of the skin after shaving has taken place and thus leaves it fresh and soft.

These also aid the hair wetting action. It has also been found that the cationic layer formed by the nitrogenised organic compounds (1) disperses the electrostatic charges of the keratinic fibres which accumulate by the rubbing of the razor over the hairs, and thus makes them more attackable by the razor. This de-electrifying action is also exercised on the bacterial membranes, to prevent the vital metabolism of the microorganisms.

The bacteriostatic action of the protective film can be possibly improved by the presence of alcohols. The non-ionic and ampholitic surface active agents (3) lower the surface tension of the composition and the hair-water interface tension, so facilitating rapid and complete contact between the applied composition and the hair to be shaved, thus facilitating and improving shaving.

In particular, as ampholitic surface active agents are particularly substantive for body proteins, they also improve the characteristics of the cationic film.

Besides facilitating the sliding of the razor over the skin, the lubricants and anti-delipidising agents (8) protect and restore the cutaneous hydrolipid film, and thus lead to an improved and more rapid restructuring of the skin after shaving.

The preserving and anti-mildew agents exert their conventional action.

Once the new and unexpected function of the primary components in the specific field of shaving had been identified, arranging the new compositions proved particularly difficult because of the extremely delicate field of application. In this respect, it was necessary to choose raw materials which were safe from the toxicological aspect, were compatible in mixture, could be formulated into solid, liquid or spray compositions, and which had a range of characteristics and compatibility sufficiently wide to enable a complete series of products to be prepared suitable for any type of skin, including dry, greasy, acne, sensitive etc.

The new compositions can be formulated as follows:

— creams or gels for preserving in jars or tubes
— powders, compressed powders, sticks
— lotions of various viscosities in containers for manual application or manual spraying

— lotions suitable for containing in cans pressurised by suitable aerosol propellents
— foam formulations for application by hand or pressure propellents
— lotions, emulsions, ointments and pastes suitable for soaking into paper, woven or non-woven pads of natural or synthetic materials, for applying or rubbing on to the skin before shaving.

One possible theoretical interpretation of the phenomena on which the present invention is based will now be given. Although plausible, this interpretation is not binding for the purposes of the invention, but represents only an attempt to clarify the scientific suppositions.

Body proteins (hair keratins, skin collagens and elastins) are known to have the classical structure of polypeptides

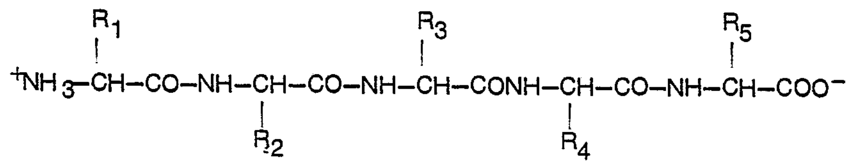

in which $R_1$, $R_2$ etc. represent various side chains of aminoacids. It is also known that chemical bonds of various kinds can be present in the protein structures:

1) hydrogen bridge bonds between the NH and CO groups,

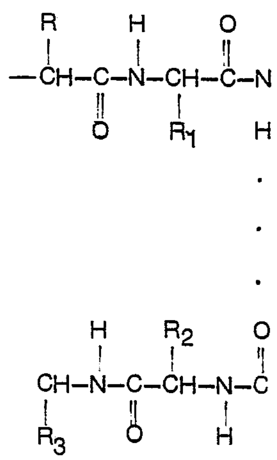

With water, these relatively simple bonds are converted into other more complicated and weaker bonds

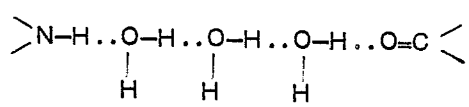

2) Saline bonds between acid and basic chains of the type:

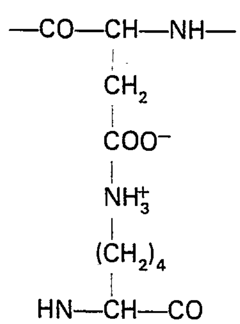

3) Sulphur bridges

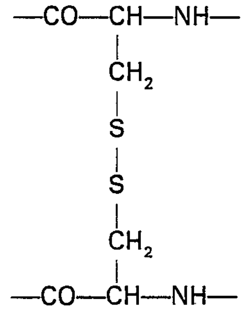

4) Hydrophobic bonds due to interaction between non-polar radicals both with themselves and with water, such interaction leading to a structure with the polar groups on the outside and the apolar chains packed inside the molecule.

If consideration is now given to the cationic organic compounds containing quaternary ammonium groups on which the present invention is based it is apparent that they contain, to a greater or lesser degree, all positively charged ammonium groups, negatively charged anionic groups, groupings capable of forming hydrogen bridges (methylene, peptide, oxyalkyl groups etc.), and long chain hydrophobic aliphatic groups.

It seems reasonable to assume that molecules of this type become disposed with their hydrophilic part towards the hydrophilic part of the skin proteins, leaving on the outside the hydrophobic chains which form a lubricating film over which the razor slides.

In addition, as already stated, as both the proteins and cationic compounds used have a great facility for forming chemical bonds, it is easy to imagine that more or less numerous chemical bonds are formed between the skin and hair on the one hand and the new shaving aids on the other, to make the film on the skin relatively stable.

As this film is rich in ionic molecular sites, it should attract the ions constituting the water molecules, and thus favour formation of hydrated protein bonds of the type indicated under point 1) above. Such hydration of the proteins implies softening of the skin, and also softening of the hair, which can thus be shaved with greater ease. The strongly polar nature of the compounds used at pH 2—7 would explain the de-electrifying action of the new products. Some typical formulations for the shaving products according to the present invention are given hereinafter by way of example only. In the examples, all the prepared compositions have been adjusted to pH 4—6, by using the acid corresponding to the anion present in the used cationic compounds.

Example 1

| Liquid shaving lotion | % by weight |
|---|---|
| * Cetyl-trimethyl ammonium chloride | 0.30 |
| * Cocoyl-amidopropyl-dimethyl-acetamide ammonium chloride | 0.50 |
| * Stearyl-pentaoxyethyl ammonium chloride | 1.00 |
| * Poly-(dimethyl-butenyl-ammonium chloride)-$\alpha$-$\omega$-bis-(triethanolammonium chloride) (Onamer M) | 3.00 |
| Polyoxyethylene (2) sorbitan-monooleate | 1.50 |
| Alkylamido betain | 0.50 |
| Laurylamine oxide | 0.30 |
| Water-soluble lanolin | 0.30 |
| Hydroxypropylcellulose | 0.50 |
| Hydrolysed Collagen M | 2.00 |

| | |
|---|---|
| Polyglycol 600 | 18.00 |
| Diisopropyladipate | 0.10 |
| Ethyl alcohol | 20.00 |
| Ter-butanol | 1.00 |
| Distilled water, paraoxybenzoic esters, colouring and perfume | to make up to 100 |

An equally effective liquid shaving lotion was prepared by replacing the asterisked components with the following mixture:

| | | |
|---|---|---|
| Laurylpyridine chloride | 0.30 | 0.30 |
| δ-Gluconamido-propyl-dimethyl-2-hydroxyethyl-ammonium chloride | 0.70 | 1.20 |
| Quaternised hydroxyethylcellulose | 0.80 | 1.20 |
| Poly-dimethyl-diallyl-ammonium chloride | 3.50 | 1.50 |

The two proportions given relate to two different alternative formulations.

These formulations when contained in a container with a manual spraying device or pressurised with propellent gas deliver foam.

By raising the ethyl alcohol to 40—60% and adding 3—8% of diethylenglycol-monomethylether or equivalent glycolic ether, similar formulations contained in a container with a manual spray device or pressurised with a propellent gas deliver lotions.

Example 2

Viscous shaving liquid suitable for packaging in various ways

| | |
|---|---|
| * Stearyl-imidazoline dioxyethyl-ammonium chloride | 0.30 |
| * Quaternium 18 Bentonite | 2.00 |
| * Lauryl-isoquinoline bromide | 0.30 |
| * Myristyl-dimethyl-ethylbenzyl-ammonium-cyclo-hexylsulphamate | 0.40 |
| Lauryl-aminopropyl-polyoxyethylene-15-amine | 1.00 |
| * Propylenecarbononate | 3.00 |
| Castor oil (40) polyoxyethylenate | 4.00 |
| Polyoxyethyleneglycol-(200)-monoctate | 3.00 |
| Polyglycol 600 | 10.00 |
| Hygroplex HHR | 3.00 |
| Diethyleneglycol-monomethylether | 5.00 |
| * Hydroxypropylcellulose | 0.30—0.80 |
| * Gum tragacanth | 0.20—0.70 |

| | |
|---|---|
| Silicone emulsion | 0.06—0.1 |
| Ethyl alcohol | 20.00 |
| 2-ethoxy-ethanol | 4.00 |
| $H_2O$, colouring, paraoxybenzoic esters, perfume | as necessary to make 100 |

Equally effective compositions were obtained by replacing the asterisked components with the following ingredients of group A or group B:

| | A | B |
|---|---|---|
| Quaternised Guar | 0.80 | 0.80 |
| Low-viscosity quaternised hydroxyethyl-cellulose | 1.65 | 1.50 |
| Reten 210 | 1.00 | — |
| Delsette 101 | — | 0.80 |
| Myristyl-pyridine chloride | 0.50 | 0.50 |
| Glycerine | 6.50 | — |
| Sorbitol | — | 7.50 |

Example 3

Shaving gel

| | |
|---|---|
| Medium viscosity hydroxypropylcellulose | 1.50 |
| * Quaternised Guar | 1.00 |
| * Dilauryl-dimethyl-ammonium chloride | 0.30 |
| * Cetil-dimethyl-hydroxyethyl-ammonium chloride | 0.30 |
| Onamer M | 2.00 |
| Nonyl-phenyl-POE(14) ether | 0.20 |
| Glycerine | 6.00 |
| Hexyleneglycol | 8.00 |
| Sorbitol | 3.00 |
| Polyoxyethylene-(20)-sorbitan-monolaurate | 0.10 |
| Amphoteric imidazoline | 0.10 |
| Elastin | 1.00 |
| Lactil | 3.00 |
| Ethyl alcohol | 35.00 |
| Distilled water, paraoxybenzoic esters, dehydro-acetic acid, colouring and perfume | to 100 |

An equally effective shaving gel was obtained by replacing only the asterisked components with the following mixture:

| | |
|---|---|
| N-(lauryl-cholamine-formly-methyl)-pyridine chloride | 0.50 |
| Alkyl-($C_{12}$—$C_{14}$)-oxyethylene-(10)-ammonium phosphate | 0.30 |
| Reten 205 | 0.30 |
| Quaternised cellulose | 1.00 |

Example 4

Product for soaking into paper and fabrics for rubbing onto the skin before shaving

| | |
|---|---|
| * Stearyl-dimethyl-benzylammonium chloride | 0.60 |
| * Quaternised polyvinylpyrrolidone | 2.00 |
| * Didecyl-dimethyl-ammonium chloride | 0.50 |
| * Onamer M | 2.00 |
| Polyoxyethylene (4) | 35.00 |
| Alkylamido betaine | 1.00 |
| Collagen | 2.00 |
| Polvyvinylpyrrolidone K30 | 1.00 |
| Polyoxyethylene-(12)-octyl-phenylether | 0.10 |
| Water soluble lanolin | 2.50 |
| Decyloleate | 0.50 |
| Ethyl alcohol | 16.50 |
| Ethyleneglycol-monophenylether | 3.00 |
| $H_2O$, colouring, paraoxybenzoic esters, perfume | to 100 |

The asterisked components can be replaced, giving an excellent product by the following mixture:

| | |
|---|---|
| Lauryl-trimethyl-ammonium chloride | 0.30 |
| Stearyl-pentaoxyethyl-ammonium chloride | 1.00 |
| Quaternised hydroxyethylcellulose | 1.00 |
| Polyoxypropylene-(9)-methyl-diethyl ammonium chloride | 2.00 |

Example 5

Powdered shaving product

| | |
|---|---|
| Quaternised hydroxyethylcellulose | 2.00 |
| * Diisobutyl-phenoxy-ethoxy-ethyl-dimethyl-benzyl-ammonium chloride in powder form | 0.50 |

| | |
|---|---|
| * Quaternium 18 Hectorite | 1.00 |
| Polyoxyethylene (20) nonophenyl-ether | 0.30 |
| Light colloidal silica | 2.00 |
| Alkylamido-betaine | 1.00 |
| Lactil | 3.50 |
| Imidazoline-urea | 0.30 |
| Paraoxybenzoic esters | 0.20 |
| Tetradecyl-octadodecanol | 1.00 |
| Talc, perfume and colouring | to 100 |

In the aforesaid composition, the asterisked components can be replaced by the following mixture to give equally good results:

| | |
|---|---|
| Lauryl-isoquinoline chloride | 0.30 |
| Stearyl-(pentaoxyethyl)-ammonium chloride | 1.20 |
| Stearyl-triethanolammonium chloride | 1.20 |
| Crotein Q (Croda G.B.) | 2.00 |
| Cartaretin F4 | 0.50 |

When compressed, these powdered mixtures give excellent shaving sticks. When made into a paste with normal mineral oils and the like, they provide excellent shaving pastes.

Example 6

Foaming base for manual shaving or for aerosol delivery

| | |
|---|---|
| Linoleic diethanolamide | 20.00 |
| Oleic diethanolamide | 20.00 |
| Alkylamido-betaine | 10.00 |
| Polyoxyethylene (10) nonyl-phenyl-ether | 3.00 |
| Glycerine | 8.00 |
| Polyethylene (20) | 6.00 |
| Hydrolised collagen M | 2.00 |
| Water soluble polyoxyethylene ether of lanolin | 5.00 |
| Caprylic-capric glycerides of polyoxyethylene(6) | 3.00 |
| * Octyl-decyl-dimethyl-ammonium chloride | 0.50 |
| * Stearyl-(pentaoxyethyl)-ammonium chloride | 2.00 |
| * Stearyl-dimethyl-hydroxyethyl-ammonium chloride | 0.50 |

| | |
|---|---|
| * Quaternised hydroxyethylcellulose | 1.50 |
| Polyvinyl pyrrolidone | 1.00 |
| Distilled water, preservatives, colouring and perfume | to 100 |

Equally good results are obtained in this composition by replacing the mixture of asterisked components with the following mixture in the proportions indicated:

| | |
|---|---|
| Quaternised hydroxyethylcellulose | 2.00 |
| Medium viscosity hydroxyethylcellulose | 0.40 |
| Distearyl-dimethyl-ammonium chloride | 1.50 |
| Dilauryl-dimethyl-ammonium chloride | 0.20 |
| Quaternised Guar | 0.70 |

Example 7

Cream shaving emulsion

| | |
|---|---|
| Non-ionic self-emulsifying glyceryl-monostearate (Teginacid X of Goldschmidt, Germany) | 12.00—16.00 |
| Vaseline oil | 1.00 |
| Lanolin oil | 0.50 |
| Octyldodecanol | 3.00 |
| Polyoxyethylene-(10)-octyl-phenyl-ether | 1.00 |
| Stearyl-heptonate | 2.00 |
| Alkylamidoamine oxide | 1.00 |
| Water soluble lanolin | 1.00 |
| Glycerine | 20.00 |
| * Stearyl-dimethyl-benzyl-ammonium chloride | 0.50 |
| * Quaternised polyvinyl pyrrolidone | 2.00 |
| * Stearyl-(pentaoxyethyl)-ammonium chloride | 1.00 |
| * Quaternised hydroxyethylcellulose | 1.00 |
| Polyoxyethylene-(20)-sorbitan-monolaurate | 3.00 |
| Hydrolised elastin | 2.00 |
| High viscosity hydroxyethylcellulose | 0.50 |
| Paraoxybenzoic esters, sorbic acid, imidazolidineurea, colouring, perfume and distilled water | to 100 |

In the aforesaid composition the asterisked components can be replaced by the following mixture:

| | |
|---|---|
| Acrylamide/poly-(dimethyl-diallyl)-ammonium chloride copolymer | 1.00 |

| | |
|---|---|
| Poly-(dimethyl-butenyl-ammonium chloride)-$\alpha$-$\omega$-bis-(triethanolammonium) chloride | 3.00 |
| N-lauryl-N-ethyl-morpholine ethoxysulphate | 0.50 |
| Low viscosity hydroxyethylcellulose | 0.80 |

The results were equally good when using emulsified shaving milks of varying viscosities and of composition analogous to the emulsified creams stated heretofore, these being obtained by adding to the compositions thus prepared 1—5% of high HLB surface active agents of polyoxyethylene-(30,40)-lauryl-ether(2—5%-type and ricinoleic alcohol ethoxylated with 60—100 moles of ethylene oxide (1—3%), together with a reduction in the Tegonacid X to 4—8%.

**Claims**

1. Cosmetic and dematological compositions as aids in shaving the skin, free of soaps, anionic polymers and anionic surfactants, having a pH between 2 and 7 and comprising as essential components:

(a) 0.5—25% of at least one cationic organic compound containing at least one quaternary ammonium nitrogen

(b) Animal and/or vegetable proteins and/or derivatives of proteins selected from the group of: soluble native collagen from animal tissues, desamido collagen, collagen complexed with cetyl-trimethyl ammonium-chloride, hydrolized animal proteins, protein hydrating products, hydrolyzed soya and milk proteins, protein derivatives from connective tissues of bovine source

(c) 0.01—15% of non ionic or anpholitic surfactants

(d) 1—50% of vetting, hydrating and supergreasing agents

beside variable amounts of other not essential components.

2. Cosmetic and dermatological compositions according to claim 1, wherein the not essential components are present in the following proportions:

| | |
|---|---|
| (e) protective and gelling colloids | 0.5 —10% |
| (f) preservatives | 0.05— 3% |
| (g) anti-foaming agents | 0.05— 3% |
| (h) lubricants and anti-delipidising agents | 0.5 —20% |

The remaining to make up the composition to 100 being of solvents and/or dispersing agents and/or solubilising agents and/or inert, diluent, filler, perfume and colouring materials.

3. Cosmetic and dermatological compositions according to claim 1, wherein the cationic organic compounds are chosen preferably from the group consisting of: Alkyl-($C_{10}$—$C_{20}$)-dimethyl-benzyl-ammoniumsaccharinate chloride or bromide; Alkyl-($C_{12}$—$C_{14}$)-oxyethylene-(10)-ammonium phosphate; Behenyltrimethyl - ammonium chloride; Cartaretin F4; Cetyl - dimethyl - hydroxymethylammonium chloride; Cetyl-trimethyl-ammonium chloride; Cocoyl-amido-propyl-dimethyl-acetamido-ammonium chloride; Acrylamide/poly-(dimethyl-diallyl)-ammonium chloride copolymer; Crotein Q (quaternised protein); Delsette 101; Didecyldimethyl ammonium chloride; Diisobutyl-cresoxyethoxy-ethyl-dimethyl-benzyl ammonium chloride; Diisobutyl-phenoxy-ethoxy-ethyl-dimethyl benzyl ammonium chloride; Dilauryl-dimethyl ammonium chloride; Dioctyl-dimethyl ammonium chloride; Distearyl-dimethyl ammonium chloride; Dodecyl-benzyl-triethanolamine chloride; Dodecyl-dimethyl-ethylbenzyl ammonium chloride; $\delta$-gluconamido-propyl-dimethyl-2-hydroxyethyl ammonium chloride; quaternised Guar; quaternised hydroxyethylcellulose; Lauryl-dimethyl-benzyl ammonium chloride; N-(lauryl-cholo-amino-formyl-methyl)-pyridine chloride; N-lauryl-N-ethyl-morpholine-ethoxysulphate; Lauryl-isoquinoline bromide; Lauryl-isoquinoline chloride; Lauryl-pyridine chloride; Lauryl-trimethyl ammonium chloride; Myristyl-amidopropyl-diethyl-2-hydroxyethyl ammonium chloride; Myristyl-pyridine chloride; Myristyl-dimethyl-ethylbenzyl-ammonium-cyclohexyl-sulphamate; Octyl-decyl-dimethyl ammonium chloride; Octyl-decyl-dimethyl ammonium chloride; Poly-(dimethyl-butenyl-ammonium chloride)$\alpha$-$\omega$-bis-(triethanolammonium) chloride (Onamer M); Poly-(dimethyldiallyl)-ammonium chloride; Polyoxypropylene (9) methyldiethyl ammonium chloride; Quaternised polyvinylpyrrolidone; Quaternium 18-Bentonite; Quaternium 18-Hectorite; Reten 205; Reten 210; Reten 220; N-(stearyl-choloamino-formyl-methyl)-pyridine chloride; Stearyl-dimethyl-benzylammonium chloride; Stearyl dimethyl-hydroxyethyl-ammonium chloride; Stearyl-imidazoline-dioxyethyl-ammonium chloride; Stearyl-pentaoxyethyl-ammonium chloride; Stearyl-triethanolammonium chloride; Trimethyl-dodecyl-benzyl ammonium chloride; Cetrex; Quaternum 29; Quaternum 54.

**Patentansprüche**

1. Kosmetische und dermatologische Zusammensetzungen als Hilfen beim Rasieren der Haut, die frei von Seifen, anionischen Polymeren und anionischen Tensiden sind, einen pH-Wert zwischen 2 und 7 besitzen und als wesentliche Bestandteile enthalten:

(a) 0,5—25% zumindest einer kationischen organischen Verbindung mit einem Gehalt an zumindest einem quaternären Ammoniumstickstoff,

(b) tierische und/oder pflanzliche Proteine und/oder Derivate von Proteinen ausgewählt aus der Gruppe von löslichem nativen Kollagen aus Tiergeweben, Desamidkollagen, Kollagen im Komplex mit Cetyltrimethylammoniumchlorid, hydrolysierten Tierproteinen, Proteinhydratisierungsprodukten, hydrolysierten Soja- und Milchproteinen, Proteinderivaten aus Bindegeweben von Rinderursprung,

(c) 0,01—15% nicht-ionische oder ampholytische Tenside,

(d) 1—50% Netz-, Hydratisierungs- und Überfettungsmittel neben variablen Anteilen an anderen nicht wesentlichen Bestandteilen.

2. Kosmetische und dermatologische Zusammensetzungen nach Anspruch 1, worin die nichtwesentlichen Bestandteile in den folgenden Anteilen vorhanden sind:

| | |
|---|---|
| (e) Schutz- und Gelierkolloide | 0,5 —10% |
| (f) Konservierungsmittel | 0,05— 3% |
| (g) Antischaummittel | 0,05— 3% |
| (h) Schmier- und Antientfettungsmittel | 0,5 —20% |

wobei der Rest der Zusammensetzung auf 100 Lösungsmittel und/oder Dispergiermittel und/oder löslichmachende Mittel und/oder inerte Materialien, Verdünnungsmittel, Füllstoffe, Parfum- und Farbstoffe sind.

3. Kosmetische und dermatologische Zusammensetzungen nach Anspruch 1, worin die kationischen organischen Verbindungen vorzugsweise aus folgender Gruppe ausgewählt sind: Alkyl-($C_{10}$—$C_{20}$)-dimethylbenzylammoniumsaccharinat-chloride oder -bromid; Alkyl-($C_{12}$—$C_{14}$)-oxyäthylen-(10)-ammoniumphosphat; Behenyltrimethylammoniumchlorid; Cartaretin F4; Cetyldimethylhydroxyäethylammoniumchlorid; Cetyltrimethylammoniumchlorid; Kokoylamidopropyldimethylacetamidoammoniumchlorid; Acrylamid/Poly-(dimethyldiallyl)-ammoniumchlorid-copolymer; Crotein Q (quaternisiertes Protein); Delsette 101; Didecyldimethylammoniumchlorid; Diisobutylcresoxyäthoxyäthyldimethylbenzylammoniumchlorid; Diisobutylphenoxyäthoxyäthyldimethylbenzylammoniumchlorid; Dilauryldimethylammoniumchlorid; Dioctyldimethylammoniumchlorid; Distearyldimethylammoniumchlorid; Dodecylbenzyltriäthanolammoniumchlorid; Dodecyldimethyläthylbenzylammoniumchlorid; $\gamma$-Gluconamidopropyldimethyl-2-hydroxyäthylammoniumchlorid; quaternisiertes Guar; quaternisierte Hydroxyäthylzellulose; Lauryldimethylbenzylammoniumchlorid; N-(Laurylcholaminoformylmethyl)-pyridinchlorid; N-Lauryl-N-äthylmorpholinäthoxysulfat; Laurylisochinolinbromid; Laurylisochinolinchlorid; Laurylpyridinchlorid; Lauryltrimethylammoniumchlorid; Myristylamidopropyldiäthyl-2-hydroxyäthylammoniumchlorid; Myristylpyridinchlorid; Myristyldimethyläthylbenzylammoniumcyclohexylsulfamat; Octyldecyldimethylammoniumchlorid; Octyldecyldimethylammoniumchlorid; Poly-(dimethylbutenylammoniumchlorid)-$\alpha$-$\omega$-bis(triäthanolammonium)chlorid (Onamer M); Poly-(dimethyldiallyl)-ammoniumchlorid; Polyoxypropylen-(9)-methyldiäthylammoniumchlorid; quaternisiertes Polyvinylpyrrolidon; Quaternium-18-Bentonite; Quaternium-18-Hectorite; Reten 205; Reten 210; Reten 220; N-(Stearylcholaminoformylmethyl)-pyridinchlorid; Stearyldimethylbenzylammoniumchlorid; Stearyldimethylhydroxyäthylammoniumchlorid; Stearylimidazolindioxyäthylammoniumchlorid; Stearylpentaoxyäthylammoniumchlorid; Stearyltriäthanolammoniumchlorid; Trimethyldodecylbenzylammoniumchlorid; Catrex; Quaternum 29; Quaternum 54.

**Revendications**

1. Compositions cosmétiques et dermatologiques destinées à servir de produits auxiliaires de rasage, exemptes de savons, de polymères anioniques et d'agents tensioactifs anioniques, ayant un pH compris entre 2 et 7, caractérisées en ce qu'elles comprennent, en tant que constituants essentiels:

(a) 0,5 à 25% d'au moins un composé organique cationique, contenant au moins un azote d'ammonium quaternaire,

(b) 1 à 20% de protéines animales et/ou végétales et/ou de dérivés de protéines choisis dans le groupe suivant: collagène natif soluble de tissus animaux, collagène désamidé, collagène complexé avec du chlorure de cétyltriméthyl-ammonium, protéines animales hydrolysées, produits d'hydratation

de protéines, protéines du soja et du lait hydrolysées, dérivés de protéines de tissus conjonctifs d'origine bovine.

(c) 0,01 à 15% d'agents tensioactifs non ioniques ou ampholitiques,

(d) 1 à 50% d'agents mouillants, hydratants et surgraissants, outre des quantités variables d'autres constituants non essentiels.

2. Compositions cosmétiques et dermatologiques selon la revendication 1, caractérisées en ce que les constituants non essentiels sont présents dans les proportions suivantes:

| | |
|---|---|
| (e) Colloïdes de protection et de gélification | 0,5 —10% |
| (f) Agents de conservation | 0,05 — 3% |
| (g) Agents anti-mousse | 0,05— 3% |
| (h) Lubrifiants et agents anti-délipidation | 0,5 —20% |

le reste en q.s.p. 100 de la composition étant fait de solvants et/ou d'agents de dispersion et/ou d'agents de solubilisation et/ou de matières inertes, diluantes, de charge, parfumantes et colorantes.

3. Compositions cosmétiques et dermatologiques selon la revendication 1, caractérisées en ce que les composés organiques cationiques sont choisis de préférence dans le groupe constitué par: des saccharinates, chlorures ou bromures d'alcoyl($C_{10}$—$C_{20}$)-diméthyl-benzyl-ammonium; des phosphates d'alcoyl($C_{12}$—$C_{14}$)-oxyéthylène-(10)-ammonium; le chlorure de béhényl-triméthyl-ammonium; le Cartaretin F4; le chlorure de cétyl-diméthylhydroxyéthyl-ammonium; le chlorure de cétyl-triméthyl-ammonium; le chlorure de cocoyl-amido-propyl-diméthyl-acétamido-ammonium; des copolymères d'acrylamide/chlorure de poly-(diméthyldially)-ammonium; le Crotein Q (protéine transformée en dérivé quaternaire); le Delsette 101; le chlorure de dodécyl-diméthyl-ammonium; le chlorure de diisobutyl-crésoxyéthoxy-éthyl-diméthyl-benzyl-ammonium; le chlorure de diisobutylphénoxy-éthoxy-éthyl-diméthyl-benzyl-ammonium; le chlorure de dilauryl-diméthyl-ammonium; le chlorure de dioctyl-diméthyl-ammonium; le chlorure de distéaryl-diméthyl-ammonium; le chlorure de dodécyl-benzyl-triéthanolammonium; le chlorure de dodécyl-diméthyl-éthyl-benzyl-ammonium; le chlorure de $\gamma$-glucon-amido-propyl-diméthyl-2-hydroxyéthyl-ammonium; le guar transformé en dérivé quaternaire; l'hydroxy-éthylcellulose transformée en dérivé quaternaire; le chlorure de lauryl-diméthyl-benzyl-ammonium; le chlorure de N-(lauryl-choloaminoformyl-méthyl)-pyridine; l'éthoxysulfate de N-lauryl-N-éthyl-morpholine; le bromure de lauryl-isoquinoléine; le chlorure de lauryl-isoquinoléine; le chlorure de lauryl-pyridine; le chlorure de lauryl-triméthyl-ammonium; le chlorure de myristylamidopropyl-diéthyl-2-hydroxyéthyl-ammonium; le chlorure de myristyl-pyridine; le cyclohexyl-sulfamate de myristyl-diméthyl-éthylbenzyl-ammonium; le chlorure d'octyl-décyl-diméthyl-ammonium; le chlorure de poly-(chlorure de diméthyl-butényl-ammonium)-$\alpha$-$\omega$-bis(triéthanolammonium) (Onamer M); le chlorure de poly-(diméthyl-diallyl)-ammonium; le chlorure de polyoxypropylène-(9)-méthyl-diéthyl-ammonium; la polyvinyl-pyrrolidone transformée en dérivé quaternaire; le Quaternium 18-Bentonite; le Quaternium 18-Hectorite; le Reten 205; le Reten 210; le Reten 220; le chlorure de N-(stéaryl-choloamino-formyl-méthyl)-pyridine; le chlorure de stéaryl-diméthyl-benzyl-ammonium; le chlorure de stéaryl-diméthylhydroxyéthyl-ammonium; le chlorure de stéaryl-imidazoline-dioxyéthyl-ammonium; le chlorure de stéaryl-pentaoxyéthyl-ammonium; le chlorure de stéaryl-triéthanol-ammonium; le chlorure de triméthyl-dodécyl-benzyl-ammonium; le Catrex; le Quaternum 29; le Quaternum 54.